# FASCICULE DE BREVET EUROPEEN

(11) **EP 0 997 134 B1**
(45) Date de publication et mention de la délivrance du brevet: **14.11.2001**
(21) Numéro de dépôt: 99402383.6
(22) Date de dépôt: 30.09.1999
(51) Int. Cl.: A61K 7/02, A61K 7/021, A61K 7/027, A61K 7/00, A61K 7/48, C09C 1/40, C09C 1/00, C09C 1/30, C09C 1/36, C09C 1/04

(54) **Utilisation d'une phase particulaire spécifique dans une composition cosmétique**
Verwendung von einer spezifische Teilchenphase in kosmetische Mitteln
Use of a specific particulate phase in a cosmetic composition

(30) Priorité: 06.10.1998 FR 9812502
(43) Date de publication de la demande: 03.05.2000
(73) Titulaire: L'OREAL, 75008 Paris (FR)
(72) Inventeur: Simon, Jean-Christophe, 75012 Paris (FR)
(74) Mandataire: Casalonga, Axel

(56) Documents cités:
- EP-A- 0 651 991
- FR-A- 2 673 372
- US-A- 5 082 660
- THURN-MÜLLER ET AL.: "Transparent colors" SEIFEN, ÖLE, FETTE, WACHSE, vol. 117, no. 20, 12 décembre 1991, AUGSBURG, DE, pages 775-778, XP000240385

## Description

La présente invention a pour objet l'utilisation d'une phase particulaire spécifique dans une composition cosmétique, notamment de maquillage, dans le but de limiter, diminuer ou supprimer les défauts des matières kératiniques, en particulier la peau, et/ou d'en remodeler les contours, ainsi qu'une composition cosmétique, notamment de maquillage, contenant une telle phase particulaire.

On cherche depuis longtemps des charges et/ou pigments apportant un effet de flou, qu'on appelle aussi "soft focus", pour uniformiser la peau et en cacher les imperfections telles que les rides ou les stries apparaissant sur les lèvres lorsqu'on applique un rouge à lèvres. Ces charges et/ou pigments ont souvent des architectures multicouches complexes telles que celles décrites dans les brevets KR 9103105-B, KR 91 03103-B, KR 91 03076-B et KR 91 03074-B ou sont des particules de forme sphérique telles que celles décrites dans le brevet FR-A-2 673 372 ou dans les brevets US 5.082.660, 5.288.481 et 5.320.834. Ces charges de l'art antérieur, lorsqu'elles sont sous forme lamellaire ou sous forme de plaquettes, possèdent un pouvoir couvrant notable qui donne à la peau un aspect non naturel. Quant aux charges de forme sphérique, elles ont un effet de diffusion faible conduisant à un masquage insuffisant des imperfections, ce qui implique la nécessité d'en utiliser une forte concentration, d'où le risque d'un produit sec et peu confortable.

Les inventeurs ont découvert avec surprise un nouveau type de phase particulaire permettant, grâce à la forme de ses particules, d'obtenir des effets optiques originaux, notamment d'apporter un effet de flou et ainsi d'estomper les imperfections de la peau et des autres matières kératiniques et/ou d'en remodeler les contours.

Les particules de cette phase particulaire sont caractérisées par le fait qu'elles comportent au moins deux faces consécutives dont les normales au point médian forment entre elles un angle au moins égal à 90° et par le fait qu'elles ont des dimensions moyennes allant de 5 à 100 microns.

L'invention a donc pour objet l'utilisation d'une phase particulaire comprenant des particules de dimensions moyennes allant de 5 à 100 microns et comportant au moins deux faces consécutives dont les normales au point médian forment entre elles un angle au moins égal à 90°, dans une composition cosmétique, notamment de maquillage, dans le but de limiter, diminuer ou supprimer les défauts des matières kératiniques et/ou de modifier la perception du volume de certaines régions desdites matières.

Selon un mode de réalisation préféré de l'invention, les particules comportent au moins deux faces consécutives planes non parallèles et sont donc de forme polyédrique.

L'angle formé par les normales au point médian de deux faces consécutives a de préférence une valeur allant de 90 à 175°. Ainsi, dans le cas de deux faces planes consécutives non parallèles, l'angle au sommet séparant les deux faces planes est au plus égal à 90°. Il varie de préférence de 5° à 90°.

Les particules de la phase particulaire utilisée selon l'invention ont des dimensions moyennes allant de préférence de 20 à 50 microns.

Les particules de la phase particulaire utilisée selon l'invention ont pour particularité de réfracter, diffuser et réfléchir la lumière incidente en la canalisant pour produire une luminosité optimale et des effets optiques originaux. Elles permettent par exemple d'illuminer les ombres du visage pour y faire disparaître les rides, ou de cacher les stries apparaissant sur les lèvres lorsqu'on applique un rouge à lèvres. L'effet de flou obtenu peut aussi permettre d'affiner ou d'augmenter le volume de certaines parties du visage et du cou et par exemple de remodeler les contours du visage. La phase particulaire joue le rôle de charge et/ou de pigment.

Les particules de la phase particulaire utilisée selon l'invention ont un indice de réfraction supérieur à 1, de préférence allant de 1,25 à 1,9 et plus préférentiellement de 1,45 à 1,55.

La nature des particules n'est pas déterminante. La transparence est cependant un critère important pour obtenir les propriétés optiques recherchées. Il peut s'agir d'une phase particulaire polymérique, constituée par exemple de polyéthylène téréphtalate (PET), de polyéthylène, notamment haute densité (PEHD), de polypropylène, de polytétrafluoréthylène (PTFE), de polychlorotétrafluoréthylène (PCTFE), de polybutylène téréphtalate (PBT), de polycarbonates, de polyuréthanes, de polythio-uréthanes, de polymères vinyliques, de vinylidène ou styréniques, comme les polyacrylates de vinyle ou vinylidène, de polymères polyacryliques et polyméthacryliques, de polythio-acrylates et polythiométhacrylates, de résines de silicone transparentes, de leurs mélanges et copolymères et de tout autre polymère biocompatible classiquement utilisé dans les lentilles, notamment ophtalmiques.

La phase particulaire utilisée selon l'invention peut également être constituée par des oxydes métalliques tels que par exemple les oxydes d'aluminium, hafnium, silicium, titane, tungstène, vanadium, zinc et zirconium. Ces oxydes doivent bien entendu être taillés selon les plans cristallins et notamment selon les plans de maille triclinique, rhomboédrique, monoclinique, mais aussi cubique, quadratique ou orthorhombique, ou obtenus par croissance cristalline et présenter de telles mailles.

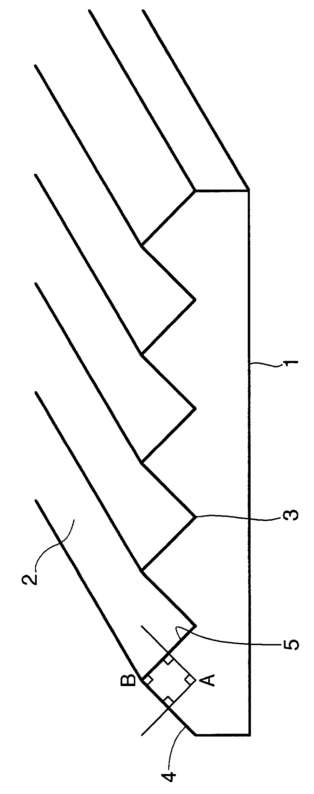

A titre d'exemple, la phase particulaire utilisée selon l'invention peut être obtenue par broyage d'une feuille de résine acrylique modifiée, de format A4, de référence BEF II (Brightness Enhancement Film II) commercialisée par la société 3M, comportant une face plane 1 et une face 2 présentant des stries 3 régulièrement disposées, telle que représentée sur la figure unique annexée. Ici, l'angle A formé par les normales à deux faces consécutives 4,5 non parallèles est de 90° environ; il s'ensuit que l'angle au sommet B entre les faces 4 et 5 est de 90° environ.

La présente invention a également pour objet une composition cosmétique, et notamment de maquillage, comprenant la phase particulaire définie ci-dessus, dans un support cosmétiquement acceptable.

La phase particulaire est présente dans la composition cosmétique selon l'invention à raison de 0,5 à 30% en poids, et de préférence 2 à 15% en poids, par rapport au poids total de la composition.

La composition selon l'invention trouve une application particulièrement intéressante dans le domaine du soin et/ou du maquillage de la peau du visage, du cou et du corps, des fibres kératiniques, des ongles, des muqueuses et/ou des semi-muqueuses des êtres humains. On entend notamment par muqueuses la partie interne de la paupière inférieure; par semi-muqueuses, on entend plus particulièrement les lèvres du visage. Par fibres kératiniques, on entend notamment les cils, les sourcils et les cheveux.

Les compositions cosmétiques selon l'invention peuvent se présenter sous la forme d'un produit de maquillage, en particulier un fond de teint, un fard à joues ou à paupières, un eye-liner, un mascara, un produit anticernes ou un rouge à lèvres, d'un produit de soin ou d'un produit solaire.

Les compositions selon l'invention peuvent se présenter sous forme fluide, gélifiée, semi-solide, solide ou pâte souple.

Elles peuvent se présenter en particulier sous forme d'émulsion simple ou complexe (H/E, E/H, H/E/H ou E/H/E), telle qu'une crème, un lait ou un gel-crème, de gel aqueux, hydroalcoolique ou anhydre plus ou moins solide, de sérum, de poudre, de pâte, de bâtonnet solide et éventuellement être conditionnées en aérosol et se présenter sous forme de mousse ou de spray.

Les compositions de l'invention peuvent être préparées selon des techniques bien connues de l'homme du métier, en particulier celles destinées à la préparation d'émulsions huile-dans-eau (H/E) ou eau-dans-huile (E/H).

Lorsqu'il s'agit d'une émulsion, la phase aqueuse de celle-ci peut comprendre une dispersion vésiculaire non-ionique préparée selon des procédés connus (Bangham, Standish and Watkins, J. Mol. Biol. 13, 238 (1965), FR-A-2 315 991 et FR-A-2 416 008). Cette dispersion peut notamment servir de dispersant de la phase huileuse.

Les compositions de maquillage, en particulier de fonds de teint, se présentent souvent sous forme de crème plus ou moins fluide comprenant une phase grasse, une phase aqueuse et une phase particulaire généralement composée de charges et/ou de pigments.

La phase grasse de la composition cosmétique selon l'invention comprend de préférence au moins une huile de silicone, volatile ou non.

A titre d'exemples d'huiles de silicones utilisées dans l'invention, on peut citer :
- les silicones volatiles cycliques ayant de 3 à 8 atomes de silicium et de préférence 4 à 6, comme par exemple les cyclométhicones telles que le cyclotétradiméthylsiloxane, le cyclopentadiméthylsiloxane (D5) ou le cyclohexadiméthylsiloxane (D6), et les produits vendus sous les dénominations : "DC FLUID 244", "DC FLUID 245", "DC FLUID 344" et "DC FLUID 345" par la société DOW CORNING ainsi que ceux vendus sous les dénominations "ABIL K4" par la société GOLDSCHMIDT, sous les dénominations "SILBIONE 70045 V2" et "SILBIONE HUILE 70045 V5" par la société RHONE POULENC ainsi que sous les dénominations "VOLATIL SILICONE 7158" et "VOLATIL SILICONE 7207" par la société UNION CARBIDE ;
- les cyclocopolymères du type diméthylsiloxane/ méthylalkylsiloxane, tels que la "silicone FZ 3109", vendue par la Société UNION CARBIDE, qui est un cyclocopolymère diméthylsiloxane/méthyloctylsiloxane;
- les silicones volatiles linéaires ayant de 2 à 9 atomes de silicium, comportant éventuellement une chaîne alkyle pendante ou en bout de chaîne en C₂-C₁₀, par exemple l'hexaméthyldisiloxane, l'hexylheptaméthyltrisiloxane et l'octylheptaméthyltrisiloxane;
- les polyalkylsiloxanes à groupements terminaux triméthylsilyle, de préférence ceux dont la viscosité à 25°C est inférieure ou égale à 0,06 m²/s, parmi lesquels on peut citer les polydiméthylsiloxanes linéaires, et notamment ceux vendus sous la dénomination "DOW CORNING FLUID 200" par la Société DOW CORNING, les alkylméthylpolysiloxanes tels que la cétyldiméthicone (dénomination CTFA), ainsi que les produits vendus sous les dénominations "AK" par la société WACKER, "SF" par la société GENERAL ELECTRIC et "ABIL" par la société GOLDSCHMIDT, tels que le produit "ABIL 10" ;
- les huiles de silicone phénylées comme les phényltriméthicones et les phényldiméthicones;
et leurs mélanges.

Les huiles de silicones volatiles, et en particulier les cyclométhicones, sont préférées pour être utilisées dans l'invention.

L'huile de silicone utilisée selon l'invention est de préférence présente dans une proportion d'au moins 5% en poids, et de préférence de 25 à 95% en poids, par rapport au poids total de la composition.

La composition selon l'invention peut également comprendre d'autres composés siliconés tels que les gommes de silicone et les cires de silicone.

Les gommes de silicone utilisables dans la composition de l'invention peuvent être des polysiloxanes de masse moléculaire élevée, de l'ordre de 200 000 à 1 000 000 et de viscosité supérieure à 500 000 mPa.s. Elles peuvent être utilisées seules ou en mélange avec un solvant tel qu'une huile polydiméthylsiloxane ou polyphénylsiloxane, ou une cyclométhicone.

Les cires de silicone utilisables dans la composition selon l'invention peuvent être des polysiloxanes linéaires substitués. On peut citer, par exemple, les cires de silicone polyéther, les alkyl ou alkoxydiméthicones ayant de 16 à 45 atomes de carbone.

Les compositions selon l'invention peuvent également comprendre des corps gras non siliconés dont des corps gras pâteux, des gommes, des cires et des huiles d'origine végétale, minérale, animale ou synthétique.

On peut définir les composés gras pâteux à l'aide d'au moins l'une des propriétés physico-chimiques suivantes :
- une viscosité de 0,1 à 40 Pa.s (1 à 400 poises), mesurée à 40°C avec un viscosimètre rotatif CONTRAVES TV équipé d'un mobile MS-r3 ou MS-r4 à la fréquence de 60 Hz,
- un point de fusion de 25-70°C, de préférence 25-55°C.

Comme cires utilisables dans l'invention, on peut citer les cires d'origine animale comme la lanoline, la cire d'abeilles, le spermaceti, les dérivés de la lanoline tels que les alcools de lanoline, la lanoline hydrogénée, hydroxylée ou acétylée, les acides gras de la lanoline et l'alcool de lanoline acétylée ; les cires d'origine végétale telles que la cire de Carnauba, de Candellila, de kapok, d'Ouricury, de riz, de jojoba hydrogénée, d'Alfa, du Japon ou les cires de fibres de liège ou de canne à sucre ou encore le beurre de cacao ; les cires minérales par exemple de paraffine, de montan, de lignite, de pétrolatum, de vaseline ou les cires microcristallines, la cérésine, l'ozokérite ; les cires synthétiques comme les cires de polyéthylène, les cires obtenues par synthèse de Fischer-Tropsch et les esters linéaires résultant de la réaction d'un acide carboxylique saturé en C₁₀ à C₄₀ et d'un alcool saturé en C₁₀ à C₄₀ comme le myristate de myristyle. On peut aussi utiliser les lanolates ou stéarates de calcium et l'huile de coprah ou de jojoba hydrogénée.

La phase grasse peut aussi comprendre une ou des huile(s) hydrocarbonée(s) ou fluorée(s).

Comme huile hydrocarbonée, on citera: toute huile (ou mélange d'huiles) fluide, stable à la température d'utilisation habituelle des produits cosmétiques, pharmaceutiques, hygiéniques, telle que les huiles d'origine végétale ou animale, minérale ou synthétique, les triglycérides d'acides gras en C₁₂ à C₁₈.

Parmi les huiles d'origine végétale ou animale, modifiées ou non, on peut citer par exemple l'huile d'amande douce, l'huile d'avocat, l'huile de ricin, l'huile d'olive, l'huile de jojoba, l'huile de tournesol, l'huile de germes de blé, l'huile de sésame, l'huile d'arachide, l'huile de pépins de raisin, l'huile de soja, l'huile de colza, l'huile de carthame, l'huile de coprah, l'huile de maïs, l'huile de noisette, le beurre de karité, l'huile de palme, l'huile de noyau d'abricot, l'huile de calophyllum, le perhydrosqualène.

Parmi les huiles d'origine minérale, on peut citer par exemple l'huile de paraffine et l'huile de vaseline.

Parmi les huiles synthétiques, on peut citer notamment les esters d'acides gras comme le myristate d'isopropyle, le palmitate d'isopropyle, le palmitate de 2-éthylhexyle, l'huile de Purcellin (octanoate de stéaryle), l'isononanoate d'isononyle ou d'isostéaryle, le lanolate d'isopropyle, les acides gras, comme les acides oléique, palmitique, stéarique, béhénique, linoléique et linolénique, les isoparaffines volatiles ou non comme les isoparaffines en C₈-C₁₆ et les polyisobutènes.

La composition selon l'invention contient de préférence une isoparaffine volatile.

A titre d'isoparaffines volatiles, on peut citer plus particulièrement l'isododécane, l'isodécane et l'isohexadécane, notamment celles vendues sous les dénominations commerciales "ISOPAR S" et "PERMETHYL 99".

On peut aussi utiliser des alcools gras en C₁₂ à C₁₈ tels que l'alcool oléique, l'alcool cétylique et l'alcool stéarylique.

En outre, la composition selon l'invention peut comprendre un ou plusieurs agents tensio-actifs et/ou une phase aqueuse et/ou un ou plusieurs agents épaississants.

La phase aqueuse peut renfermer des adjuvants communément utilisés dans les gels aqueux et les émulsions cosmétiques. En outre, la phase aqueuse peut comprendre de 0,5 % à 20 % en poids, par rapport au poids total de la phase aqueuse, d'un monoalcool inférieur en C₂-C₆ et/ou d'un polyol tel que le glycérol, le butylèneglycol, l'isoprèneglycol, le propylèneglycol.

Les tensio-actifs sont utilisés dans des émulsions notamment à des teneurs allant de 0,5 à 30 %, de préférence de 0,5 à 10 % en poids, par rapport au poids total de l'émulsion.

L'agent épaississant peut être choisi parmi les argiles modifiées telles que le silicate de magnésium modifié (bentone gel VS38 de RHEOX), l'hectorite modifiée par le chlorure de distéaryl diméthyl ammonium (bentone 38 CE de RHEOX), les acides polyacryliques réticulés et les gommes de guar et celluloses modifiées ou non.

Les compositions selon l'invention peuvent comprendre également des matières colorantes. Ces matières colorantes peuvent être soit des pigments minéraux ou organiques ou des laques, qui sont généralement insolubles dans les milieux aqueux et organiques, soit des colorants solubles dans les milieux aqueux ou organiques.

Les pigments additionnels doivent être de nature et/ou utilisés à des concentrations ne masquant peu ou pas l'effet de flou ou "soft focus" recherché. Ils peuvent être blancs ou colorés, minéraux et/ou organiques. On peut citer, parmi les pigments minéraux, les dioxydes de titane, de zirconium ou de cérium, ainsi que les oxydes de zinc, de fer ou de chrome sous forme de particules sphériques, le bleu ferrique, les nacres telles que le mica recouvert d'oxyde de titane, d'oxyde de fer, de pigment naturel ou d'oxychlorure de bismuth ainsi que le mica titane coloré. Parmi les pigments organiques, on peut citer le noir de carbone, et les laques de baryum, strontium, calcium, aluminium. Les pigments peuvent également présenter une surface hydrophobe ou peuvent être traités de manière à rendre leur surface hydrophobe; ce traitement peut être effectué selon les méthodes connues de l'homme du métier ; les pigments peuvent notamment être enrobés par des composés siliconés tels que des PDMS et/ou des polymères, notamment des polyéthylènes et/ou des acides aminés.

Ces pigments additionnels sont de préférence présents à des taux inférieurs à 15% en poids par rapport au poids total de la composition.

Les colorants peuvent être des colorants hydrosolubles tels que le sel disodique de ponceau, le sel disodique du vert d'alizarine, le sel trisodique d'amarante, le sel disodique de tartrazine, le sel monosodique de rhodamine, le sel disodique de fuchsine, la xanthophylle, le bleu de méthylène ou le jus de betterave, ou encore des colorants liposolubles comme le carotène, le rouge Soudan, le brun Soudan, D & C Red n°17, D & C Orange n°5, D& C Yellow n°11, l'huile de soja.

Les colorants sont généralement présents dans les compositions à une teneur inférieure à 15%, de préférence allant de 0,1 à 10 % en poids, par rapport au poids total de la composition.

La composition selon l'invention peut également comprendre un composé filmogène.

Le composé filmogène peut être choisi parmi les polymères en dispersion aqueuse, comme par exemple des polymères acryliques, polyesters et/ou polyuréthannes en dispersion aqueuse. Par exemple la composition peut comprendre un copolymère acétate de vinyle/p-tertio-butyl-benzoate de vinyle/acide crotonique en dispersion aqueuse partiellement neutralisé, stabilisé.

La composition selon l'invention peut également comprendre une dispersion de particules de polymère dans un milieu non aqueux, comme décrit par exemple dans le document EP-A-749 747.

La composition selon l'invention peut comprendre en outre tous additifs usuellement utilisés dans le domaine cosmétique, tels que des antioxydants, des parfums, des huiles essentielles, des conservateurs, des actifs cosmétiques ou dermatologiques comme les hydratants, les vitamines, les sphingolipides tels que les céramides, les filtres solaires, ou des polymères liposolubles. Bien entendu, l'homme du métier veillera à choisir ce ou ces éventuels composés complémentaires, et/ou leur quantité, de manière telle que les propriétés avantageuses de la composition selon l'invention, et notamment l'effet de flou, ne soient pas, ou substantiellement pas, altérées par l'adjonction envisagée. Ces additifs peuvent être présents dans la composition à raison de 0 à 15% en poids.

L'invention a également pour objet un procédé de traitement non thérapeutique des matières kératiniques, notamment un procédé de maquillage, consistant à appliquer sur lesdites matières une composition telle que définie ci-dessus afin de limiter, diminuer ou supprimer les imperfections de ces matières et/ou de modifier la perception du volume de certaines régions de ces dernières.

L'invention sera mieux illustrée à l'aide de l'exemple suivant.

### Exemple : Emulsion de fond de teint E/H

- Mélange de polyméthylcétyl diméthyl méthyl 9 g siloxane oxyéthyléné oxypropyléné, polyglycéryl-4-isostéarate et hexyl laurate vendu sous la dénomination commerciale "Abil WE 09" par la société Goldschmidt
- mélange stéarate de glycol acétylé et tristéarine vendu 0,5 g sous la dénomination commerciale "Unitwix" par la société Guardian
- cyclométhicone D₅/D₆ 25 g
- diphényl diméthicone 6 g
- isododécane 4,55 g
- hectorite 4 g
- phase particulaire préparée par broyage 10 g d'une feuille plastique BEF II commercialisée par la société 3M
- copolymère acétate de vinyle/p-tertio-butyl-benzoate 20 g de vinyle/acide crotonique en dispersion aqueuse partiellement neutralisé, stabilisé
- adipate de diisopropyle 1 g
- eau q.s.p. 100 g

L'émulsion est obtenue selon le procédé de préparation suivant :
- on prédisperse le pigment dans une partie de la cyclométhicone.
- on homogénéise le reste d'huile avec les tensio-actifs à 40-50°C ;
- on laisse refroidir. On ajoute à ce mélange le pigment et l'hectorite modifiée prégonflée dans un peu d'isododécane ;
- on ajoute l'ensemble de la phase aqueuse dans la phase grasse précédente, sous agitation lente d'abord, puis à forte puissance pendant 10 minutes ;
- on ajoute, sous agitation lente, le copolymère et l'adipate de diisopropyle.

Ce fond de teint a une texture crémeuse, légère et douce. Son application sur la peau est facile et donne à la peau un aspect lisse en masquant les imperfections et les rides. Le toucher est très doux. Le confort est bon.

## Revendications

1. Utilisation d'une phase particulaire comprenant des particules de dimensions moyennes allant de 5 à 100 microns et comportant au moins deux faces consécutives dont les normales au point médian forment entre elles un angle au moins égal à 90°, dans une composition cosmétique, notamment de maquillage, dans le but de limiter, diminuer ou supprimer les défauts des matières kératiniques et/ou de modifier la perception du volume de certaines régions desdites matières.

2. Utilisation selon la revendication 1, **caractérisée par le fait que** l'angle formé par les normales au point médian de deux faces consécutives a une valeur allant de 90 à 175°.

3. Utilisation selon la revendication 1 ou 2, **caractérisée par le fait que** les particules comportent au moins deux faces consécutives planes non parallèles.

4. Utilisation selon la revendication 3, **caractérisée par le fait que** l'angle au sommet séparant deux faces planes consécutives non parallèles est au plus égal à 90°.

5. Utilisation selon la revendication 4, **caractérisée par le fait que** l'angle au sommet séparant deux faces planes consécutives non parallèles a une valeur allant de 5 à 90°.

6. Utilisation selon l'une quelconque des revendications précédentes, **caractérisée par le fait que** les particules ont des dimensions moyennes allant de 20 à 50 microns.

7. Utilisation selon l'une quelconque des revendications précédentes, **caractérisée par le fait que** les particules ont un indice de réfraction supérieur à 1 et de préférence allant de 1,25 à 1,9.

8. Utilisation selon la revendication 7, **caractérisée par le fait que** les particules ont un indice de réfraction allant de 1,45 à 1,55.

9. Utilisation selon l'une quelconque des revendications précédentes, **caractérisée par le fait que** la phase particulaire est de nature polymérique et est constituée de polyéthylène téréphtalate, de polyéthylène, de polypropylène, de polytétrafluoréthylène, de polychlorotétrafluoréthylène, de polybutylène téréphtalate, de polycarbonates, de polyuréthanes, de polythio-uréthanes, de polymères vinyliques, de vinylidène ou styréniques, de polymères polyacryliques et polyméthacryliques, de polythio-acrylates et polythiométhacrylates, de résines de silicone transparentes, de leurs mélanges et copolymères.

10. Utilisation selon l'une quelconque des revendications 1 à 8, **caractérisée par le fait que** la phase particulaire est constituée par des oxydes métalliques choisis parmi les oxydes d'aluminium, hafnium, silicium, titane, tungstène, vanadium, zinc et zirconium.

11. Utilisation selon l'une quelconque des revendications précédentes, **caractérisée par le fait que** la phase particulaire est présente dans la composition cosmétique à raison de 0,5 à 30 % en poids et de préférence 2 à 15 % en poids, par rapport au poids total de la composition.

12. Utilisation selon l'une quelconque des revendications précédentes, **caractérisée par le fait que** la composition cosmétique est un produit de maquillage choisi parmi un fond de teint, un fard à joues ou à paupières, un eye-liner, un mascara, un produit anticernes ou un rouge à lèvres.

13. Utilisation selon l'une quelconque des revendications précédentes, **caractérisée par le fait que** la composition cosmétique est une émulsion dont la phase grasse comprend au moins une huile de silicone.

14. Utilisation selon la revendication 13, **caractérisée par le fait que** l'huile de silicone est une huile de silicone volatile, de préférence une cyclométhicone.

15. Utilisation selon la revendication 13 ou 14, **caractérisée par le fait que** l'huile de silicone est présente dans une proportion d'au moins 5 % en poids, et de préférence de 25 à 95 % en poids, par rapport au poids total de la composition.

16. Utilisation selon l'une quelconque des revendications 13 à 15, **caractérisée par le fait que** la phase grasse comprend en outre une isoparaffine volatile.

17. Composition cosmétique, notamment de maquillage, **caractérisée par le fait qu'**elle comprend, dans un support cosmétiquement acceptable, une phase particulaire constituée de particules de dimensions moyennes allant de 5 à 100 microns et comportant au moins deux faces consécutives dont les normales au point médian forment entre elles un angle au moins égal à 90°.

18. Composition cosmétique selon la revendication 17, **caractérisée par le fait que** l'angle formé par les normales au point médian de deux faces consécutives a une valeur allant de 90 à 175°.

19. Composition cosmétique selon la revendication 17 ou 18, **caractérisée par le fait que** les particules comportent au moins deux faces consécutives planes non parallèles.

20. Composition cosmétique selon la revendication 19, **caractérisée par le fait que** l'angle au sommet séparant deux faces planes consécutives non parallèles est au plus égal à 90°.

21. Composition cosmétique selon la revendication 20, **caractérisée par le fait que** l'angle au sommet séparant deux faces planes consécutives non parallèles a une valeur allant de 5 à 90°.

22. Composition cosmétique selon l'une quelconque des revendications 17 à 21, **caractérisée par le fait que** les particules ont des dimensions moyennes allant de 20 à 50 microns.

23. Composition cosmétique selon l'une quelconque des revendications 17 à 22, **caractérisée par le fait que** les particules ont un indice de réfraction supérieur à 1 et de préférence allant de 1,25 à 1,9.

24. Composition cosmétique selon la revendication 23, **caractérisée par le fait que** les particules ont un indice de réfraction allant de 1,45 à 1,55.

25. Composition cosmétique selon l'une quelconque des revendications 17 à 24, **caractérisée par le fait que** la phase particulaire est constituée d'un polymère choisi parmi le polyéthylène téréphtalate, le polyéthylène, le polypropylène, le polytétrafluoréthylène, le polychlorotétrafluoréthylène, le polybutylène téréphtalate, les polycarbonates, les polyuréthanes, les polythiouréthanes, les polymères vinyliques, de vinylidène ou styréniques, les polymères polyacryliques et polyméthacryliques, les polythio-acrylates et polythiométhacrylates, les résines de silicone transparentes, leurs mélanges et copolymères.

26. Composition cosmétique selon l'une quelconque des revendications 17 à 24, **caractérisée par le fait que** la phase particulaire est constituée par des oxydes métalliques choisis parmi les oxydes d'aluminium, hafnium, silicium, titane, tungstène, vanadium, zinc et zirconium.

27. Composition cosmétique selon l'une quelconque des revendications 17 à 26, **caractérisée par le fait que** la phase particulaire est présente à raison de 0,5 à 30 % en poids, et de préférence 2 à 15 % en poids, par rapport au poids total de la composition.

28. Composition cosmétique selon l'une quelconque des revendications 17 à 27, **caractérisée par le fait qu'**elle constitue un produit de maquillage choisi parmi un fond de teint, un fard à joues ou à paupières, un eye-liner, un mascara, un produit anticernes ou un rouge à lèvres.

29. Composition cosmétique selon l'une quelconque des revendications 17 à 28, **caractérisée par le fait qu'**elle se présente sous forme d'émulsion simple ou complexe, de gel aqueux, hydro-alcoolique ou anhydre, de sérum, de poudre, de pâte, de bâtonnet solide ou est conditionnée en aérosol et se présente sous forme de mousse ou de spray.

30. Composition cosmétique selon l'une quelconque des revendications 17 à 29, **caractérisée par le fait qu'**elle comprend une phase grasse comprenant au moins une huile de silicone.

31. Composition cosmétique selon la revendication 30, **caractérisée par le fait que** l'huile de silicone est présente dans une proportion d'au moins 5 % en poids, et de préférence de 25 à 95 % en poids, par rapport au poids total de la composition.

32. Composition cosmétique selon la revendication 30 ou 31, **caractérisée par le fait que** l'huile de silicone est une huile de silicone volatile, et de préférence une cyclométhicone.

33. Composition cosmétique selon l'une quelconque des revendications 17 à 32, **caractérisée par le fait qu'**elle comprend en outre des corps gras non siliconés choisis parmi les corps gras pâteux, les gommes, les cires et les huiles d'origine végétale, minérale, animale ou synthétique.

34. Composition cosmétique selon la revendication 33, **caractérisée par le fait qu'**elle contient une isoparaffine volatile.

35. Composition cosmétique selon l'une quelconque des revendications 17 à 34, **caractérisée par le fait qu'**elle comprend en outre une phase aqueuse, et/ou un ou plusieurs agents tensio-actifs et/ou un ou plusieurs agents épaississants.

36. Composition cosmétique selon l'une quelconque des revendications 17 à 35, **caractérisée par le fait qu'**elle comprend en outre des matières colorantes choisies parmi les pigments minéraux ou organiques, les laques et les colorants solubles dans les milieux aqueux ou organiques.

37. Composition cosmétique selon l'une quelconque des revendications 17 à 36, **caractérisée par le fait qu'**elle comprend en outre des additifs choisis parmi les antioxydants, les parfums, les huiles essentielles, les conservateurs, les actifs cosmétiques ou dermatologiques, les polymères liposolubles et les dispersions de particules de polymères dans un milieu aqueux ou non aqueux.

38. Procédé de traitement non thérapeutique des matières kératiniques, notamment procédé de maquillage, **caractérisé par le fait qu'**il consiste à appliquer sur les matières kératiniques une composition cosmétique selon l'une quelconque des revendications 17 à 37, dans le but de limiter, diminuer ou supprimer les imperfections de ces matières et/ou de modifier la perception du volume de certaines régions desdites matières.

## Patentansprüche

1. Verwendung einer partikelförmigen Phase, die Partikel enthält, deren mittlere Größe im Bereich von 5 bis 100 µm liegt und die mindestens zwei aufeinander folgende Flächen aufweisen, deren Flächennormalen am Medianpunkt miteinander einen Winkel von mindestens 90 ° bilden, in einer kosmetischen Zusammensetzung, insbesondere Schminkzusammensetzung, mit dem Ziel, die Unvollkommenheiten der Keratinmaterialien zu begrenzen, zu verringern oder zu beseitigen und/oder die Wahrnehmung des Volumens bestimmter Bereiche dieser Materialien zu modifizieren.

2. Verwendung nach Anspruch 1, **dadurch gekennzeichnet, daß** der Winkel, den die Flächennormalen der beiden benachbarten Flächen am Medianpunkt miteinander bilden, einen Wert im Bereich von 90 bis 175 ° hat.

3. Verwendung nach Anspruch 1 oder 2, **dadurch gekennzeichnet, daß** die Partikel mindestens zwei nicht parallele, ebene, aufeinander folgende Flächen aufweisen.

4. Verwendung nach Anspruch 3, **dadurch gekennzeichnet, daß** der Kantenwinkel, der zwei nicht parallele, aufeinander folgende, ebene Flächen trennt, einen Wert von höchstens 90 ° aufweist.

5. Verwendung nach Anspruch 4, **dadurch gekennzeichnet, daß** der Kantenwinkel, der zwei nicht parallele, aufeinander folgende, ebene Flächen trennt, einen Wert im Bereich von 5 bis 90 ° aufweist.

6. Verwendung nach einem der vorhergehenden Ansprüche, **dadurch gekennzeichnet, daß** die Partikel eine mittlere Größe im Bereich von 20 bis 50 µm aufweisen.

7. Verwendung nach einem der vorhergehenden Ansprüche, **dadurch gekennzeichnet, daß** die Partikel einen Brechungsindex größer als 1 und vorzugsweise im Bereich von 1,25 bis 1,9 aufweisen.

8. Verwendung nach Anspruch 7, **dadurch gekennzeichnet, daß** die Partikel einen Brechungsindex im Bereich von 1,45 bis 1,55 aufweisen.

9. Verwendung nach einem der vorhergehenden Ansprüche, **dadurch gekennzeichnet, daß** die partikelförmige Phase ein Polymermaterial ist und aus Polyethylenterephthalat, Polyethylen, Polypropylen, Polytetrafluorethylen, Polychlortetrafluorethylen, Polybutylenterephthalat, Polycarbonten, Polyurethanen, Polythiourethanen, Vinylpolymeren, Vinyliden- oder Styrolpolymeren, Polyacrylpolymeren oder Polymethacrylpolymeren, Polythioacrylaten und Polythiomethacrylaten, durchsichtigen Siliconharzen, ihren Gemischen und/oder Copolymeren besteht.

10. Verwendung nach einem der Ansprüche 1 bis 8, **dadurch gekennzeichnet, daß** die partikelförmige Phase aus Metalloxid(en) besteht, das/die unter den Oxiden von Aluminium, Hafnium, Silicium, Titan, Wolfram, Vanadium, Zink und Zirconium ausgewählt ist/sind.

11. Verwendung nach einem der vorhergehenden Ansprüche, **dadurch gekennzeichnet, daß** die partikelförmige Phase in der kosmetischen Zusammensetzung in einem Anteil von 0,5 bis 30 Gew.-% und vorzugsweise 2 bis 15 Gew.-%, bezogen auf das Gesamtgewicht der Zusammensetzung, enthalten ist.

12. Verwendung nach einem der vorhergehenden Ansprüche, **dadurch gekennzeichnet, daß** die kosmetische Zusammensetzung ein Schminkprodukt ist, das ausgewählt ist unter Make-up, Rouge oder Lidschatten, Eyeliner, Mascara, Produkten gegen Augenringe und Lippenstiften.

13. Verwendung nach einem der vorhergehenden Ansprüche, **dadurch gekennzeichnet, daß** die kosmetische Zusammensetzung eine Emulsion ist, deren Fettphase mindestens ein Siliconöl enthält.

14. Verwendung nach Anspruch 13, **dadurch gekennzeichnet, daß** das Siliconöl ein flüchtiges Siliconöl und vorzugsweise ein Cyclometicon ist.

15. Verwendung nach Anspruch 13 oder 14, **dadurch gekennzeichnet, daß** das Siliconöl in einem Anteil von mindestens 5 Gew.-% und vorzugsweise 25 bis 95 Gew.-%, bezogen auf das Gesamtgewicht der Zusammensetzung, enthalten ist.

16. Verwendung nach einem der Ansprüche 13 bis 15, **dadurch gekennzeichnet, daß** die Fettphase außerdem ein flüchtiges Isoparaffin enthält.

17. Kosmetische Zusammensetzung, insbesondere Schminkzusammensetzung, **dadurch gekennzeichnet, daß** sie in einem kosmetisch akzeptablen Träger eine partikelförmige Phase enthält, die aus Partikeln besteht, deren mittlere Größe im Bereich von 5 bis 100 µm liegt und die mindestens zwei aufeinander folgende Flächen aufweisen, deren Flächennormalen am Medianpunkt miteinander einen Winkel von mindestens 90 ° bilden.

18. Kosmetische Zusammensetzung nach Anspruch 17, **dadurch gekennzeichnet, daß** der Winkel, den die Flächennormalen der beiden aufeinander folgenden Flächen am Medianpunkt miteinander bilden, einen Wert im Bereich von 90 bis 175 ° hat.

19. Kosmetische Zusammensetzung nach Anspruch 17 oder 18, **dadurch gekennzeichnet, daß** die Partikel mindestens zwei nicht parallele, ebene, aufeinander folgende Flächen aufweisen.

20. Kosmetische Zusammensetzung nach Anspruch 19, **dadurch gekennzeichnet, daß** der Kantenwinkel, der zwei nicht parallele, aufeinander folgende, ebene Flächen trennt, einen Wert von höchstens 90 ° aufweist.

21. Kosmetische Zusammensetzung nach Anspruch 20, **dadurch gekennzeichnet, daß** der Kantenwinkel, der zwei nicht parallele, aufeinander folgende, ebene Flächen trennt, einen Wert im Bereich von 5 bis 90 ° aufweist.

22. Kosmetische Zusammensetzung nach einem der Ansprüche 17 bis 21, **dadurch gekennzeichnet, daß** die Partikel eine mittlere Größe im Bereich von 20 bis 50 µm aufweisen.

23. Kosmetische Zusammensetzung nach einem der Ansprüche 17 bis 22, **dadurch gekennzeichnet, daß** die Partikel einen Brechungsindex größer als 1 und vorzugsweise im Bereich von 1,25 bis 1,9 aufweisen.

24. Kosmetische Zusammensetzung nach Anspruch 23, **dadurch gekennzeichnet, daß** die Partikel einen Brechungsindex im Bereich von 1,45 bis 1,55 aufweisen.

25. Kosmetische Zusammensetzung nach einem der Ansprüche 17 bis 24, **dadurch gekennzeichnet, daß** die partikelförmige Phase aus einem Polymer besteht, das unter Polyethylenterephthalat, Polyethylen, Polypropylen, Polytetrafluorethylen, Polychlortetrafluorethylen, Polybutylenterephthalat, Polycarbonten, Polyurethanen, Polythiourethanen, Vinylpolymeren, Vinyliden- oder Styrolpolymeren, Polyacrylpolymeren oder Polymethacrylpolymeren, Polythioacrylaten oder Polythiomethacrylaten, transparenten Siliconharzen, ihren Gemischen und/oder Copolymeren ausgewählt ist.

26. Kosmetische Zusammensetzung nach einem der Ansprüche 17 bis 24, **dadurch gekennzeichnet, daß** die partikelförmige Phase aus Metalloxid(en) besteht, das/die unter den Oxiden von Aluminium, Hafnium, Silicium, Titan, Wolfram, Vanadium, Zink und Zirconium ausgewählt ist/sind.

27. Kosmetische Zusammensetzung nach einem der Ansprüche 17 bis 26, **dadurch gekennzeichnet, daß** die partikelförmige Phase in der kosmetischen Zusammensetzung in einem Anteil von 0,5 bis 30 Gew.-% und vorzugsweise 2 bis 15 Gew.-%, bezogen auf das Gesamtgewicht der Zusammensetzung, enthalten ist.

28. Kosmetische Zusammensetzung nach einem der Ansprüche 17 bis 28, **dadurch gekennzeichnet, daß** sie ein Schminkprodukt darstellt, das ausgewählt ist unter Make-up, Rouge oder Lidschatten, Eyeliner, Mascara, Produkten gegen Augenringe und Lippenstiften.

29. Kosmetische Zusammensetzung nach einem der Ansprüche 17 bis 28, **dadurch gekennzeichnet, daß** sie in Form einer einfachen oder komplexen Emulsion, eines wäßrigen, wäßrig-alkoholischen oder wasserfreien Gels, eines Serums, eines Pulvers, einer Paste, eines festen Stifts vorliegt oder als Aerosol verpackt ist und in Form eines Schaumes oder Sprays vorliegt.

30. Kosmetische Zusammensetzung nach einem der Ansprüche 17 bis 29, **dadurch gekennzeichnet, daß** sie eine Fettphase enthält, die mindestens ein Siliconöl enthält.

31. Kosmetische Zusammensetzung nach Anspruch 30, **dadurch gekennzeichnet, daß** das Siliconöl in einem Anteil von mindestens 5 Gew.-% und vorzugsweise 25 bis 95 Gew.-%, bezogen auf das Gesamtgewicht der Zusammensetzung, enthalten ist.

32. Kosmetische Zusammensetzung nach einem der Ansprüche 30 und 31, **dadurch gekennzeichnet, daß** das Siliconöl ein flüchtiges Siliconöl und vorzugsweise ein Cyclometicon ist.

33. Kosmetische Zusammensetzung nach einem der Ansprüche 17 bis 32, **dadurch gekennzeichnet, daß** sie außerdem nicht siliconhaltige Fettsubstanzen enthält, die unter pastösen Fettsubstanzen, Gummen, Wachsen und Ölen pflanzlicher, mineralischer, tierischer oder synthetischer Herkunft ausgewählt sind.

34. Kosmetische Zusammensetzung nach Anspruch 33, **dadurch gekennzeichnet, daß** sie ein flüchtiges Isoparaffin enthält.

35. Kosmetische Zusammensetzung nach einem der Ansprüche 17 bis 34, **dadurch gekennzeichnet, daß** sie außerdem eine wäßrige Phase und/oder ein oder mehrere grenzflächenaktive Stoffe und/oder ein oder mehrere Verdickungsmittel enthält.

36. Kosmetische Zusammensetzung nach einem der Ansprüche 17 bis 35, **dadurch gekennzeichnet, daß** sie außerdem Färbemittel enthält, die unter anorganischen oder organischen Pigmenten, Lacken und und in wäßrigen oder organischen Medien löslichen Farbstoffen ausgewählt sind.

37. Kosmetische Zusammensetzung nach einem der Ansprüche 17 bis 36, **dadurch gekennzeichnet, daß** sie außerdem Additive enthält, die unter Antioxidantien, Parfüms, etherischen Ölen, Konservierungsmitteln, kosmetischen oder dermatologischen Wirkstoffen, fettlöslichen Polymeren und Dispersionen von Polymerpartikeln in wäßrigem oder nicht-wäßrigem Medium ausgewählt sind.

38. Verfahren zur nicht-therapeutischen Behandlung von Keratinmaterialien, insbesondere Schminkverfahren, **dadurch gekennzeichnet, daß** es darin besteht, auf die Keratinmaterialien eine kosmetische Zusammensetzung nach einem der Ansprüche 17 bis 37 aufzutragen, mit dem Ziel, die Unvollkommenheiten der Keratinmaterialien zu begrenzen, zu verringern oder zu beseitigen und/oder die Wahrnehmung des Volumens bestimmter Bereiche dieser Materialien zu modifizieren.

## Claims

1. Use of a particulate phase comprising particles with mean dimensions ranging from 5 to 100 microns and comprising at least two consecutive faces, the normals of which at the median point form, between one another, an angle at least equal to 90°, in a cosmetic composition, in particular a make-up composition, for the purpose of limiting, decreasing or eliminating the blemishes of keratinous substances and/or of modifying the perception of the volume of certain regions of the said substances.

2. Use according to Claim 1, **characterized in that** the angle formed by the normals at the median point of two consecutive faces has a value ranging from 90 to 175°.

3. Use according to Claim 1 or 2, **characterized in that** the particles comprise at least two nonparallel flat consecutive faces.

4. Use according to Claim 3, **characterized in that** the angle at the vertex separating two nonparallel flat consecutive faces is at most equal to 90°.

5. Use according to Claim 4, **characterized in that** the angle at the vertex separating two nonparallel flat consecutive faces has a value ranging from 5 to 90°.

6. Use according to any one of the preceding claims, **characterized in that** the particles have mean dimensions ranging from 20 to 50 microns.

7. Use according to any one of the preceding claims, **characterized in that** the particles have a refractive index of greater than 1 and preferably ranging from 1.25 to 1.9.

8. Use according to Claim 7, **characterized in that** the particles have a refractive index ranging from 1.45 to 1.55.

9. Use according to any one of the preceding claims, **characterized in that** the particulate phase is polymeric in nature and is composed of poly(ethylene terephthalate), of polyethylene, of polypropylene, of polytetrafluoroethylene, of polychlorotetrafluoroethylene, of poly(butylene terephthalate), of polycarbonates, of polyurethanes, of polythiourethanes, of vinyl, vinylidene or styrene polymers, of polyacrylic and polymethacrylic polymers, of polythioacrylates and polythiomethacrylates, of transparent silicone resins, or of their mixtures and copolymers.

10. Use according to any one of Claims 1 to 8, **characterized in that** the particulate phase is composed of metal oxides chosen from aluminium, hafnium, silicon, titanium, tungsten, vanadium, zinc and zirconium oxides.

11. Use according to any one of the preceding claims, **characterized in that** the particulate phase is present in the cosmetic composition in a proportion of 0.5 to 30% by weight and preferably 2 to 15% by weight with respect to the total weight of the composition.

12. Use according to any one of the preceding claims, **characterized in that** the cosmetic composition is a make-up product chosen from a foundation, a face powder, an eyeshadow, an eyeliner, a mascara, a concealer or a lipstick.

13. Use according to any one of the preceding claims, **characterized in that** the cosmetic composition is an emulsion, the fatty phase of which comprises at least silicone oil.

14. Use according to Claim 13, **characterized in that** the silicone oil is a volatile silicone oil, preferably a cyclomethicone.

15. Use according to Claim 13 or 14, **characterized in that** the silicone oil is present in a proportion of at least 5% by weight and preferably of 25 to 95% by weight with respect to the total weight of the composition.

16. Use according to any one of Claims 13 to 15, **characterized in that** the fatty phase additionally comprises a volatile isoparaffin.

17. Cosmetic composition, in particular a make-up composition, **characterized in that** it comprises, in a cosmetically acceptable vehicle, a particulate phase composed of particles with mean dimensions ranging from 5 to 100 microns and comprising at least two consecutive faces, the normals of which at the median point form, between one another, an angle at least equal to 90°.

18. Cosmetic composition according to Claim 17, **characterized in that** the angle formed by the normals at the median point of two consecutive faces has a value ranging from 90 to 175°.

19. Cosmetic composition according to Claim 17 or 18, **characterized in that** the particles comprise at least two nonparallel flat consecutive faces.

20. Cosmetic composition according to Claim 19, **characterized in that** the angle at the vertex separating two nonparallel flat consecutive faces is at most equal to 90°.

21. Cosmetic composition according to Claim 20, **characterized in that** the angle at the vertex separating two nonparallel flat consecutive faces has a value ranging from 5 to 90°.

22. Cosmetic composition according to any one of Claims 17 to 21, **characterized in that** the particles have mean dimensions ranging from 20 to 50 microns.

23. Cosmetic composition according to any one of Claims 17 to 22, **characterized in that** the particles have a refractive index of greater than 1 and preferably ranging from 1.25 to 1.9.

24. Cosmetic composition according to Claim 23, **characterized in that** the particles have a refractive index ranging from 1.45 to 1.55.

25. Cosmetic composition according to any one of Claims 17 to 24, **characterized in that** the particulate phase is composed of a polymer chosen from poly(ethylene terephthalate), polyethylene, polypropylene, polytetrafluoroethylene, polychlorotetrafluoroethylene, poly(butylene terephthalate), polycarbonates, polyurethanes, polythiourethanes, vinyl, vinylidene or styrene polymers, polyacrylic and polymethacrylic polymers, polythioacrylates and polythiomethacrylates, transparent silicone resins, or their mixtures and copolymers.

26. Cosmetic composition according to any one of Claims 17 to 24, **characterized in that** the particulate phase is composed of metal oxides chosen from aluminium, hafnium, silicon, titanium, tungsten, vanadium, zinc and zirconium oxides.

27. Cosmetic composition according to any one of Claims 17 to 26, **characterized in that** the particulate phase is present in a proportion of 0.5 to 30% by weight and preferably 2 to 15% by weight with respect to the total weight of the composition.

28. Cosmetic composition according to any one of Claims 17 to 27, **characterized in that** it constitutes a makeup product chosen from a foundation, a face powder, an eyeshadow, an eyeliner, a mascara, a concealer or a lipstick.

29. Cosmetic composition according to any one of Claims 17 to 28, **characterized in that** it is provided in the form of a simple or complex emulsion, of an aqueous, aqueous/alcoholic or anhydrous gel, of a serum, of a powder, of a paste or of a solid stick or is packaged in an aerosol and is provided in the form of a foam or spray.

30. Cosmetic composition according to any one of Claims 17 to 29, **characterized in that** it comprises a fatty phase comprising at least one silicone oil.

31. Cosmetic composition according to Claim 30, **characterized in that** the silicone oil is present in a proportion of at least 5% by weight and preferably of 25 to 95% by weight with respect to the total weight of the composition.

32. Cosmetic composition according to Claim 30 or 31, **characterized in that** the silicone oil is a volatile silicone oil and preferably a cyclomethicone.

33. Cosmetic composition according to any one of Claims 17 to 32, **characterized in that** it additionally comprises non-silicone fatty substances chosen from pasty fatty substances or gums, waxes and oils of vegetable, mineral, animal or synthetic origin.

34. Cosmetic composition according to Claim 33, **characterized in that** it comprises a volatile isoparaffin.

35. Cosmetic composition according to any one of Claims 17 to 34, **characterized in that** it additionally comprises an aqueous phase and/or one or more surface-active agents and/or one or more thickening agents.

36. Cosmetic composition according to any one of Claims 17 to 35, **characterized in that** it additionally comprises colouring materials chosen from inorganic or organic pigments, lakes and dyes which are soluble in aqueous or organic media.

37. Cosmetic composition according to any one of Claims 17 to 36, **characterized in that** it additionally comprises additives chosen from antioxidants, fragrances, essential oils, preservatives, cosmetic or dermatological active principles, fat-soluble polymers and dispersions of polymer particles in an aqueous or nonaqueous medium.

38. Process for the nontherapeutic treatment of keratinous substances, in particular a make-up process, **characterized in that** it consists in applying a cosmetic composition according to any one of Claims 17,to 37 to keratinous substances for the purpose of limiting, decreasing or eliminating the imperfections of these substances and/or of modifying the perception of the volume of some regions of the said substances.
